## Europäisches Patentamt

## European Patent Office

## Office européen des brevets

(19)

(11) Veröffentlichungsnummer: **0 087 552**
**B1**

(12) # EUROPÄISCHE PATENTSCHRIFT

(45) Veröffentlichungstag der Patentschrift:
**19.04.89**

(21) Anmeldenummer: **83100079.9**

(22) Anmeldetag: **07.01.83**

(51) Int. Cl.⁴: **G 01 F 1/66,** A 61 M 1/00, A 61 B 5/14, A 61 B 5/00

(54) **Vorrichtung zur Messung der Durchflussmenge und Verwendung derselben.**

(30) Priorität: **26.02.82 CH 1216/82**

(43) Veröffentlichungstag der Anmeldung:
**07.09.83 Patentblatt 83/36**

(45) Bekanntmachung des Hinweises auf die Patenterteilung:
**19.04.89 Patentblatt 89/16**

(84) Benannte Vertragsstaaten:
**AT BE DE FR GB IT NL SE**

(56) Entgegenhaltungen:
**EP-A-0 036 658**
**DE-A-2 556 158**
**DE-A-2 607 022**
**US-A-4 147 059**

(73) Patentinhaber: **Doltron AG, Webernstrasse 5, CH-8610 Uster (CH)**

(72) Erfinder: **Rechsteiner, Alfred, Stöcklerstrasse 10, CH- 8610 Uster (CH)**

(74) Vertreter: **Blum, Rudolf Emil Ernst, c/o E. Blum & Co Patentanwälte Vorderberg 11, CH- 8044 Zürich (CH)**

EP 0 087 552 B1

LIBER, STOCKHOLM 1989

## Beschreibung

Die vorliegende Erfindung betrifft eine Vorrichtung zum Messen der durch einen Kunststoffschlauch strömenden Blutmenge mittels Ultraschall mit einem Sender, einer Sonde, die ein zweiteiliges Gehäuse in das der Kunststoffschlauch einlegbar ist und zwei Schwingungselemente aufweist, um ein dem Ausgangssignal des Senders entsprechendes Signal in das durch den Kunststoffschlauch strömende Blut einzuleiten und ein der Durchflussgeschwindigkeit proportionales Signal zu erzeugen, und einer Auswerteinrichtung, die einen Empfänger mit einem selektiven HF-Verstärker und einem nachgeschalteten Demodulator, einen Regelverstärker und einen Signalformer aufweist und die an die Sonde angeschlossen ist, um ein der Durchflussmenge proportionales Ausgangssignal zu erzeugen sowie eine Blutspendeeinrichtung mit einer solchen Vorrichtung.

Es ist bekannt, die Durchflussgeschwindigkeit von Flüssigkeiten mittels Ultraschall zu messen, wobei das Messprinzip auf dem Dopplereffekt beruht. Dies setzt aber voraus, dass eine Reflektion bewirkende Komponenten in der Flüssigkeit enthalten sind. So ist es bekannt, dass bei entgastem, reinem Wasser dieses Messprinzip nicht anwendbar ist.

Aus der DE-A-3 556 158 ist eine Vorrichtung zur Überwachung des Fliessvorganges bekannt, bei der die Reflektion durch die in der Leitung bewegten Partikeln oder die Strömungslinien in der Flüssigkeit verursacht wird.

Die Vorrichtung arbeitet mit einer Frequenz von 1 MHz. Das rote Blutkörperchen hat einen Durchmesser von 8 μm und eine Dicke von 2 μm. Die Strömungsgeschwindigkeit des Blutes ab Spender beträgt ca. 10 - 30 cm/s. Unter diesen Voraussetzungen wird mit der bekannten Vorrichtung und innerhalb des angegebenen Frequenzbereichs kein brauchbares Signal erzeugt.

Aus der EP-A1-0 036 658 ist ein Ultraschall-Messgerät zur Bestimmung der Strömung eines Mediums in einer Leitung bekannt, die eine aussen an der Leitung gehaltene Baueinheit mit zwei Ultraschall-Wandlereinrichtungen aufweist. Die Baueinheit hat zwei scharnierartig verbundene Befestigungsteile, die zwischen sich die Leitung aufnehmen und miteinander verspannt sind. Diese Baueinheit ist nicht an Kunststoffschläuche anlegbar, weil keine definierte Meßstrecke durch die Baueinheit gebildet wird.

Aus der US-A-4 147 059 ist ein Gerät bekannt, das zur Messung der Durchflussmenge von Blut in einer Leitung bestimmt ist. Dieses Gerät enthält einen Sender, der ein Ausgangssignal in der Grössenordnung von 3,13 MHz abgibt, einen Wandler, mit zwei Schwingungselementen, der ein den durch das in der Leitung strömende Blut reflektierten Schwingungen entsprechendes Ausgangssignal abgibt, einen HF-Verstärker, der das Ausgangssignal des Wandlers verstärkt und einen AM-Demodulator, um ein der Durchflussgeschwindigkeit proportionales Ausgangssignal zu erzeugen. Ferner ist ein Signalformer vorgesehen, dem ein Zähler und ein Decodierer nachgeschaltet ist, die ein der Durchflussgeschwindigkeit entsprechendes digitales Signal zwecks Anzeige abgeben.

Dabei wird davon ausgegangen, dass das Messprinzip sowohl bei Teilchengrössen, die grösser als die Wellenlänge der eingeleiteten Schwingung als auch bei Teilchengrössen die kleiner als die Wellenlänge der Schwingung sind, anwendbar ist. Da die roten Blutkörperchen einen Durchmesser von 8 μm und eine Dicke von 2 μm haben, sind diese kleiner als die Wellenlänge der 3,13 MHz, die im Blut ca. 480 μm beträgt. Die Messung soll dadurch erfolgen, dass das Blutkörperchen durch die Schwingung in Bewegung gesetzt wird und zu einem sekundären Geber wird, der als eine Punktquelle wirkt. Diese Annahme ist nicht zutreffend, weil einerseits das Blutkörperchen vom Aufbau her kein starrer Körper ist und andererseits das das Blutkörperchen umgebene Blutplasma derart dämpft, dass eine Eigenresonanz bei der Schwingung von 3,13 MHz nicht auftreten kann. Versuche, die Durchflussmenge des vom Spender direkt abgegebenen Blutes mit einem solchen Gerät extrakorporal zu messen, schlugen fehl, d.h. es wurde kein messbares Signal abgegeben.

Wird das Gerät, wie in dieser Patentschrift erwähnt, bei extrakorporalen Kreisläufen, d.h. bei Blutdialysegeräten und Herz/Lungenmaschinen zur Messung der durch die Schläuche strömenden Blutmenge verwendet, so ist dem Blut eine Flüssigkeit beigemischt, z. B. ein Gerinnungshemmungsmittel. Durch eine solche Beimischung verändert sich im Blut die Bindungskapazität der roten Blutkörperchen für gelöste Gase. Diese werden als Mikrobläschen freigesetzt und bilden ideale Echowände, die ein ausreichendes Signal-Rauschverhältnis für eine zuverlässige Messung ergeben. Dies ist durch die extrem unterschiedlichen Schallgeschwindigkeiten im Blut und in der Luft begründet.

Die der Erfindung zugrundeliegende Aufgabe ist es, eine Vorrichtung der eingangs genannten Art zu schaffen, mit der die von einem Spender abgegebene Blutmenge gemessen werden kann, um ein Gerinnungshemmungsmittel im richtigen Verhältnis dem gespendeten Blut zuführen zu können.

Diese Aufgabe wird erfindungsgemäss mit den kennzeichnenden Merkmalen des Anspruchs 1 erreicht.

Die mit der Vorrichtung erzielbaren Vorteile sind darin zu sehen, dass die Messvorrichtung mit der Flüssigkeit nicht in Kontakt kommt und handelsübliche Kunststoffschläuche verwendet werden können.

Es ist ferner von Vorteil, wenn der Signalumformer einen einstellbaren Kalibrierverstärker zu Umwandlung des Geschwindigkeitsproportionalen Signals in ein mengenproportionales Signal aufweist.

Eine Blutspendeeinrichtung mit einer Vorrichtung zum Messen der Blutmenge ist durch die Merkmale des Patentanspruches 9 gekennzeichnet.

Die dadurch erzielbaren Vorteile sind im wesentlichen darin zu sehen, dass auf die Zugabe der empirisch ermittelten und auf eine bestimmte Menge von Spenderblut abgestimmten Gerinnungshemmungsmittelmenge vor dem Spendervorgang verzichtet werden kann, dass das ideale Misbhverhältnis der beiden Komponenten während des Spendervorganges eingehalten werden kann, so dass ein Überschuss an Gerinnungshemmungsmittel zu Beginn des Spendervorganges verhindert wird und wichtige Blutbestandteile im Blut teilweise oder ganz zerstört werden und dass die Qualität und Haltbarkeit des gespendeten Blutes verbessert werden kann.

Bei einem erfindungsgemässen Ausführungsbeispiel hat das Gehäuse einen Körper mit einer länglichen Ausnehmung, in welche der Schlauch einlegbar ist und einen Deckel, um den Schlauch in der Ausnehmung zu fixieren. Dies hat den Vorteil, dass der Schlauch im Bereich der Sonde über eine bestimmte Länge gerade verläuft.

Bei einem anderen Ausführungsbeispiel sind die Schwingelemente quer zur Längsachse der Ausnehmung angeordnet.

Bei einem weiteren bevorzugten Ausführungsbeispiel sind die Schwingelemente parallel zur Längsachse der Ausnehmung angeordnet, wobei die der Ausnehmung zugewandten Flächen einen Winkel von mindestens 90° einschliessen. Dies hat den Vorteil, dass der Messbereich im wesentlichen im Zentrum des Schlauches liegt und dass ein wesentlicher Teil des eingespeisten Signals nicht reflektiert wird, so dass das N/S-Verhältnis verbessert wird.

Im folgenden sind Ausführungsbeispiele des Erfindungsgegenstandes anhand der beiliegenden Zeichnungen näher erläutert.

Es zeigen:
Fig. 1 ein Blockschema eines Ausführungsbeispiels der erfindungsgemässen Vorrichtung,
Fig. 2 ein Blockschema eines anderen Ausführungsbeispiels der erfindungsgemässen Vorrichtung,
Fig. 3 und 4 räumliche Ansichten von Ausführungsbeispielen eines Teils einer Sonde ohne Deckel,
Fig. 5 ein Schnitt durch die in Fig. 4 dargestellte Sonde mit Deckel, und
Fig. 6 ein Schema einer Einrichtung zur Entnahme einer physiologischen Flüssigkeit und Beimischung eines Gerinnungshemmungsmitteb in der die Messvorrichtung verwendet wird.

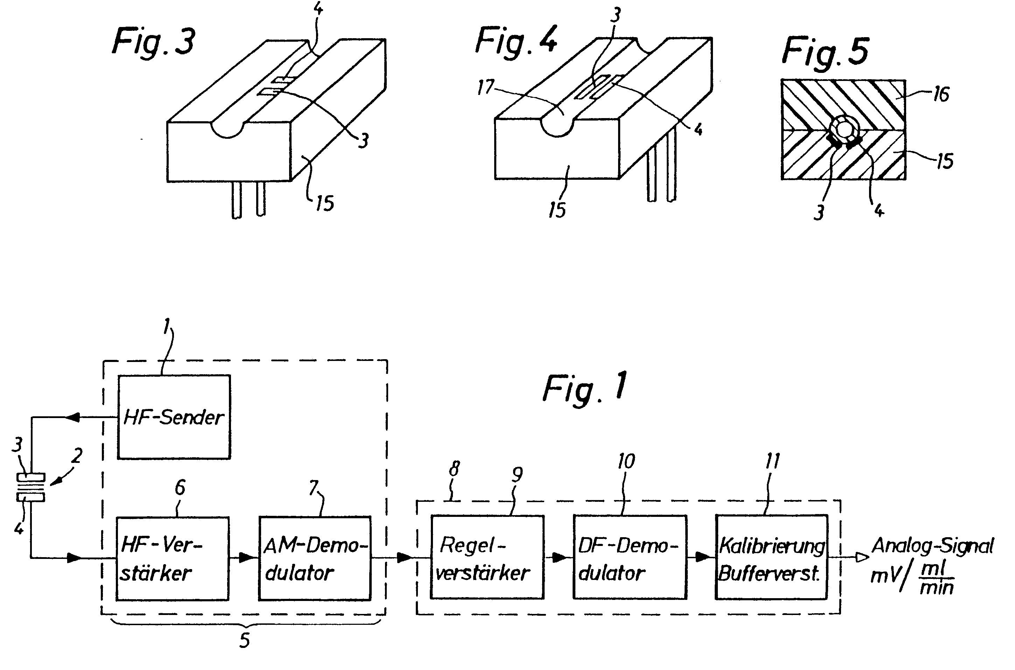

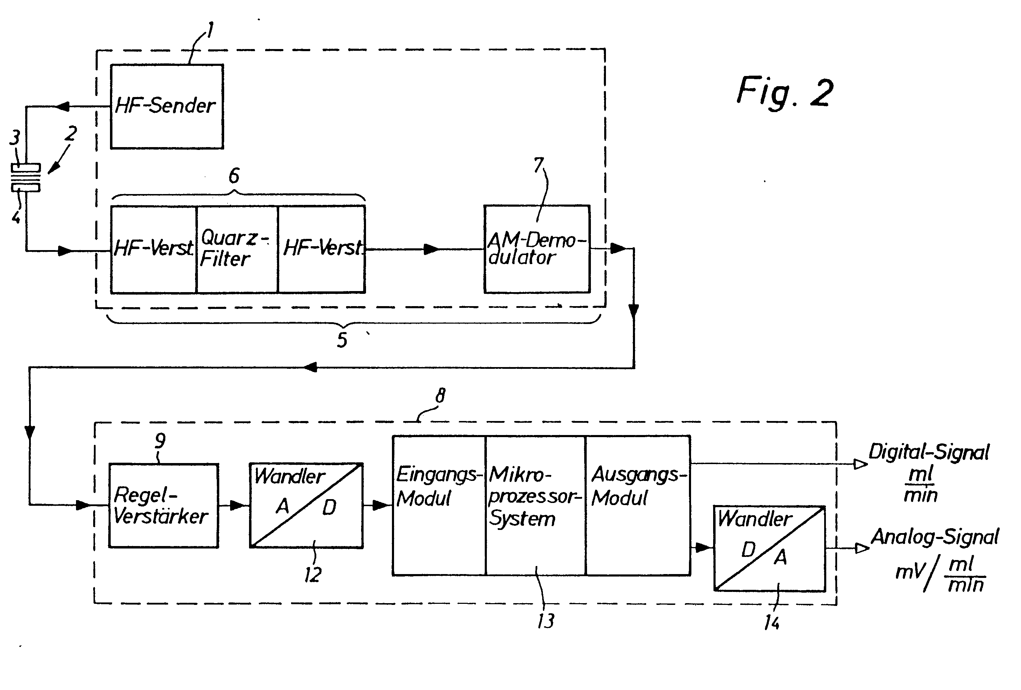

Wie die Figuren 1 und 2 zeigen enthält die Vorrichtung im wesentlichen ein Sendeglied 1, eine Sonde 2 und eine Auswerteinrichtung. Die Sonde 2 weist zwei Schwingelemente 3 und 4 auf, die mit dem Sendeglied 1, bzw. der Auswerteinrichtung elektrisch verbunden und an einen Kunststoffschlauch anlegbar sind.

Die Auswerteinrichtung nach Fig. 1 umfasst eine Demodulatoreinrichtung 5 mit einem selektiv geregelten Verstärker 6 und einem Demodulator 7, die in Serie geschaltet sind, und eine Wandlereinrichtung 8 mit einem Regelverstärker 9, einem Demodulator 10 und einem Kalibrierverstärker 11.

Die Sonde 2, Fig. 3, 4, 5 besteht aus dem Sondenkörper 15, mit der länglichen Ausnehmung 17 zur Aufnahme des Schlauches und dem Deckel 16, welcher vor allem dazu dient, den Schlauch im Sondenkörper 15 zu fixieren. Durch diese Fixation werden die in der Ausnehmung 17 beabstandet angeordneten Schwingelemente 3, 4 an die Aussenfläche des Schlauches gepresst. Für gute Schwingungsübertragung muss der Kontakt zwischen den Schwingelementen 3, 4 und der Aussenfläche des Schlauches möglichst ohne Luftspalt erfolgen.

In der Ausführung nach Fig. 3 liegen die Schwingelemente 3, 4 quer zur Längsachse der Ausnehmung 17.

In der Ausführung nach Fig. 4, 5 liegen die Schwingelemente 3, 4 parallel zur Längsachse der Ausnehmung 17. Die Schwingelemente 3, 4 sind in einem Winkel angeordnet, wodurch eine gewisse Fokussierungswirkung eintritt, derart, dass die Messung hauptsächlich in der Mitte des Schlauches stattfindet, wo die Flüssigkeitsgeschwindigkeit am grössten ist. Dies verbessert das Nutzsignal/Störsignal-Verhältnis.

Das Sendeglied 1, welches so angeordnet und temperaturkompensiert ist, dass mit den anderen Elementen der Demodulatoreinrichtung 5 temperaturgleichlauf erzielt wird, gibt ein Ausgangssignal von 9,5 MHz ab, das an das eine Schwingelement 3 der Sonde angelegt wird.

Das aus piezoelektrischem Kristall bestehende Schwingelement 3 wird durch das Signal des Sendeglieds 1 in mechanische Schwingung versetzt. Die Schwingung wird über die Schlauchwandung auf das im Schlauch strömende Medium übertragen. Sowohl am Schlauch, an der Sonde mit Deckel 15, 16 und am strömenden Medium wird diese Schwingung reflektiert. Die von den im strömenden Medium enthaltenen Partikeln reflektierte Schwingung weist eine Frequenzverschiebung auf, welche proportional zur Geschwindigkeit des strömenden Mediums ist (Doppler-Effekt).

Das zweite Schwingelement 4 der Sonde wird durch die reflektierten Schwingungen angeregt und gibt ein elektrisches Ausgangssignal ab. Der dem Schwingelement 4 nachgeschaltete Verstärker 6 bereitet das HF-Signal soweit auf, dass es im nachfolgenden Demodulator 7 demoduliert werden kann. Sowohl HF-Verstärker 6 wie Demodulator 7 sind so ausgelegt, dass für die bekannten Empfangsfrequenzen eine möglichst hohe Unterdrückung der nichtinformationshaltigen Frequenzen erfolgt. Das entstehende NF-Signal, dessen Frequenz proportional der Strömungsgeschwindigkeit ist, wird danach der Wandlereinrichtung 8 zugeführt.

In der Wandlereinrichtung 8 sorgt ein Regelverstärker 9 für eine weitere Verbesserung des NF-Signals, insbesonders in bezug auf das Nutzsignal/Störsignal-Verhältnis, wonach dieses in einem nachfolgenden Demodulator 10 in eine Gleichspannung gewandelt wird. Die Spannung ist nun proportional zur Geschwindigkeit des strömenden Mediums. Der Kalibrierverstärker 11 sorgt durch seine Einstellmöglichkeit dafür, dass das Signal linear und mengenproportional ausgegeben werden kann.

Die Auswerteinrichtung nach Fig. 2 folgt zunächst der bekannten Konfiguration nach Fig. 1, zeichnet sich aber innerhalb der Demodulatoreinrichtung 5 dadurch aus, dass der HF-Verstärker 6 zur Erhöhung der Selektivität mit Quarzfiltern versehen ist.

Die Wandlereinrichtung 8 ist wesentlich verschieden aufgebaut, indem das NF-Signal nach dem Regelverstärker 9 mit dem Analog/Digital-Wandler 12 in ein Digitalsignal umgewandelt wird. In dieser Form kann es nun durch ein Mikroprozessorsystem 13 bearbeitet werden. Neben der Wandlung von geschwindigkeitsproportionalen zu mengenproportionalen Signaleinheiten können weitere Signalbewertungen und Signalbeeinflussungen vorgenommen werden. Als Beispiele seien nur genannt: Schwell- und Maximalwerterfassung, Erfassung der Änderungen, Erzeugen von linearen oder nichtlinearen Ausgangskurven.

Nach dem Mikroprozessorsystem 13 steht das Ausgangssignal in digitaler Form oder aber nach erneuter Wandlung im Digital/Analog-Wandler wieder als Gleichspannungssignal zur Verfügung.

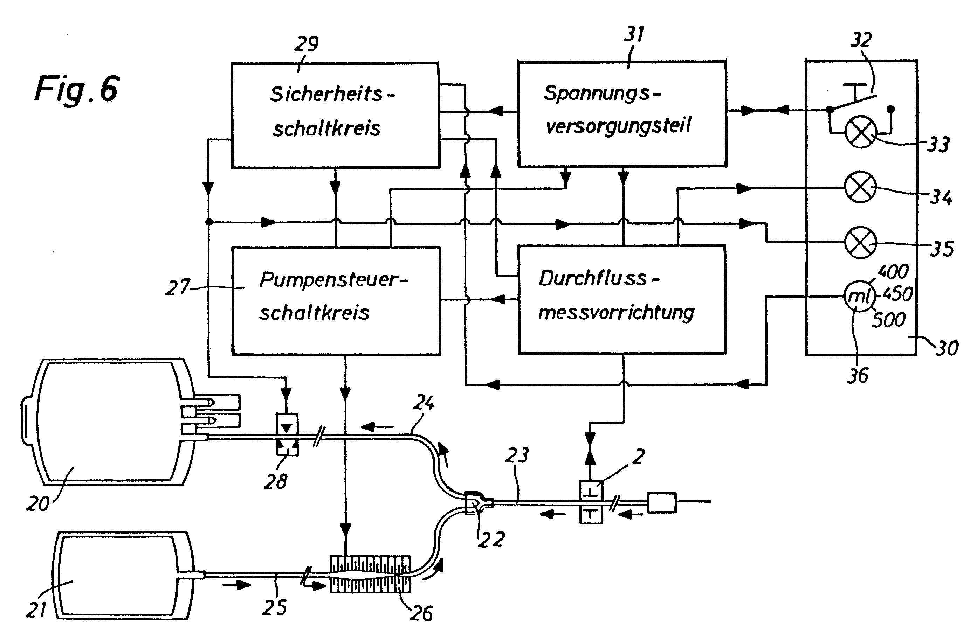

Die Fig. 6 zeigt ein Blockschema einer Einrichtung zur Blutentnahme und Beimischung eines Gerinnungshemmungsmittels mit einem Entnahmebesteck.

Das Entnahmebesteck besteht im wesentlichen aus einem ersten Beutel 20 zur Aufnahme des gespendeten Blutes, einem zweiten Beutel 21, in dem ein Gerinnungshemmungsmittel enthalten ist, und eine Schlauchanordnung, um die Verbindung zwischen Spender und dem ersten und zweiten Beutel 20, 21 herzustellen. Die Schlauchanordnung besteht aus einem Verbindungsorgan 22 und drei Kunntstoffschläuchen 23, 24, 25, die einerseits an das Verbindungsorgan 22 angeschlossen und andererseits mit dem Spender, dem ersten Beutel 20, bzw. dem zweiten Beutel 21 verbindbar sind.

Das Verbindungsorgan 22 hat einen Y-förmig ausgebildeten Durchlass, an dessen Stamm der zum Spender führende Kunststoffschlauch 23 und an dessen Zweige der zum ersten, bzw. zum zweiten Beutel führende Kunststoffschlauch 24, 25, angeschlossen sind.

Wie Fig. 6 zeigt, enthält die Einrichtung die vorstehend beschriebene Durchflussmessvorrichtung, die über die Sonde 2 an den zum Spender führende Kunststoffschlauch 23 anlegbar ist, um die gespendete Blutmenge zu messen, eine Peristaltikpumpe 26 mit einem Pumpensteuerschaltkreis 27, die an den zum zweiten Beutel 21 führenden Kunststoffschlauch 25 anlegbar ist, um das Gerinnungshemmungsmittel zuzuführen, eine Absperreinrichtung 28 mit einem Sicherheitsschaltkreis 29, einen Anzeige- und Bedienungsteil 30 und einen Spannungsversorgungsteil 31.

Die Peristaltikpumpe 26 ist bekannt und in der US-Patentschrift Nr. 4 302 164 beschrieben. Die Absperreinrichtung 28 besteht aus zwei gegeneinander bewegbaren Organen (nicht dargestellt), die bei Betätigung den Kunststoffschlauch 24 zusammendrücken und dadurch den Durchfluss unterbrechen.

Im Anzeige- und Bedienungsteil 30 ist ein Schalter 32 zum Ein- und Ausschalten der Einrichtung, eine Lampe 33 zur Anzeige des Einschaltzustandes, eine Lampe 34 zum Anzeigen, dass Blut gespendet wird, eine Lampe 35 zum Anzeigen, dass der Spendevorgang unterbrochen und beendet ist und ein Wahlschalter 36 enthalten, um die zu entnehmende Blutmenge einzustellen.

Der Spannungsversorgungsteil 31 ist mit der Durchflussmessvorrichtung, dem Pumpensteuerschaltkreis 27 und dem Sicherheitsschaltkreis 29 verbunden und gibt eine Speisespannung an diese ab.

Die Durchflussmessvorrichtung ist mit dem Pumpensteuerschaltkreis 27 und dem Sicherheitsschaltkreis 29 verbunden. Der Sicherheitsschaltkreis 29 ist seinerseits mit der Absperreinrichtung 28 und dem Pumpensteuerschaltkreis 27 verbunden.

Nach dem Einlegen des Entnahmebestecks wird die Einrichtung eingeschaltet und das Besteck an den Spender angesetzt. Zwischenzeitlich wird die Peristaltikpumpe 26 in Betrieb gesetzt und das Gerinnungshemmungsmittel bis zum Verbindungsorgan 22 gefördert. Danach wird die Peristaltikpumpe 26 abgeschaltet.

Setzt der Blutfluss ein, so gibt die Durchflussmessvorrichtung das der Durchflussmenge proportionale Signal an den Sicherheitsschaltkreis 29, um die totale Durchflussmenge zu bestimmen, und an den Pumpensteuerschaltkreis 27 ab, um die Peristaltikpumpe 28 wieder einzuschalten, und in Abhängigkeit dieses Signals zu steuern. Dadurch fördert die Peristaltikpumpe 27 eine Menge des Gerinnungshemmungsmittels, die einem vorher berechneten Verhältnis entspricht, in das Verbindungsorgan 22. Da das Gerinnungshemmungsmittel gegen den Blutstrom gefördert wird, erfolgt im Verbindungsorgan 22 eine gute Durchmischung.

Der Sicherheitsschaltkreis 29 weist Grenzwertschaltkreise (nicht dargestellt) auf, die bei Erreichen der am Schalter 36 eingestellten Menge die Absperreinrichtung 28 betätigen, so die Blutentnahme gestoppt wird, und die bei Unterschreiten der vorgewählten Durchflussmenge ein akustisches Signal auslösen, so dass festgestellt wird, dass die Blutentnahme nicht optimal verläuft.

**Patentansprüche**

1. Vorrichtung zum Messen der durch einen Kunststoffschlauch strömenden Blutmenge mittels Ultraschall mit einem Sender (1), einer Sonde (2), die ein zweiteiliges Gehäuse mit einer Ausnehmung (17), in die der Kunststoffschlauch einlegbar ist, und zwei Schwingelemente (3, 4) aufweist, um ein dem Ausgangssignal des Senders (1) entsprechendes Signal in das durch den Kunststoffschlauch strömende Blut einzuleiten und ein der Durchflussgeschwindigkeit proportionales Signal zu erzeugen, und einer Auswerteinrichtung, die einen Empfänger (5) mit einem selektiven HF-Verstärker (16) und einem nachgeschalteten Demodulator (17), einen Regelverstärker (9) und einem Signalformer (10, 11) aufweist und die an die Sonde angeschlossen ist, um ein der Durchflussmenge proportionales Ausgangssignal zu erzeugen, dadurch gekennzeichnet, dass der Sender (1) ausgebildet ist, ein Ausgangssignal mit einer Frequenz von 9,5 MHz zu erzeugen und einen temperaturkompensierten Schaltkreisaufbau aufweist, dass die Schwingelemente (3, 4) in der Sonde so angeordnet sind, dass jeweils mindestens ein Flächenabschnitt der Schwingelemente (3, 4) mit der Oberfläche der Ausnehmung (17) eine Auflagefläche für den Schlauch bildet, dass der HF-Verstärker (6) des Empfängers (5) zusammen mit dem Demodulator so ausgelegt ist, dass das N/S-Verhältnis mindestens 2 : 1 beträgt und dass der Signalformer (8) Mittel (10, 11; 13) aufweist, um das der Durchflussgeschwindigkeit proportionale Signal in ein der Durchflussmenge proportionales Signal umzuwandeln.

2. Vorrichtung nach Anspruch 1, dadurch gekennzeichnet, dass die Mittel einen Demodulator (10), um das NF-Signal in ein analoges Gleichspannungssignal umzuwandeln, und einen Kalibrierverstärker (11) aufweisen.

3. Vorrichtung nach Anspruch 1, dadurch gekennzeichnet, dass die Sonde (2) einen Körper (15) mit einer länglichen Ausnehmung (17), in die ein Abschnitt des Schlauches einlegbar ist, und einen Deckel (16) aufweist, um den Schlauchabschnitt in der Ausnehmung (17) zu fixieren.

4. Vorrichtung nach Anspruch 1, dadurch gekennzeichnet, dass die Schwingelemente (3, 4) quer zur Längsachse der Ausnehmung (17) angeordnet sind.

5. Vorrichtung nach Anspruch 1, dadurch gekennzeichnet, dass die Schwingelemente (3, 4) parallel zur Längsachse der Ausnehmung (17) angeordnet sind, wobei die der Ausnehmung zugewandten Flächen einen Winkel von mindestens 90° einschliessen.

6. Vorrichtung nach Anspruch 1, dadurch gekennzeichnet, dass der HF-Verstärker (6) des Empfängers (5) zur Erhöhung der Selektivität mit Quarzfiltern versehen ist.

7. Vorrichtung nach Anspruch 1, dadurch gekennzeichnet, die des Mittel ein Mikroprozessorsystem (13) mit einem vorgeschalteten A/D-Wandler (12) aufweisen.

8. Vorrichtung nach Anspruch 7, dadurch gekennzeichnet, dass ein D/A-Wandler (14) dem Mikroprozessorsystem (13) nachgeschaltet ist.

9. Blutspendeeinrichtung mit einer Vorrichtung nach Anspruch 1, gekennzeichnet durch einen ersten Beutel (20) zur Aufnahme des gespendeten Blutes, einen zweiten Beutel (21), der ein Gerinnungshemmungsmittel aufweist, durch eine Schlauchanordnung (22, 23 bis 25), die einerseits mit dem Spender und andererseits mit dem ersten und zweiten Beutel (20, 21) verbindbar ist, durch eine Peristaltikpumpe (26) zum Zuführen des Gerinnungshemmungsmittels, die an die Schlauchanordnung anlegbar ist, und durch eine Steuereinrichtung (27 bis 30), um in Abhängigkeit der von der Vorrichtung gemessenen Menge des gespendeten Blutes eine Menge des Gerinnungshemmungsmittels dem gespendeten Blut zuzuführen.

10. Einrichtung nach Anspruch 9, dadurch gekennzeichnet, dass das Verbindungsorgan (22) derart angeordnet ist, daß das von der Peristaltikpumpe (26) geförderte Gerinnungshemmungsmittel gegen den Strom des Blutes in den ersten Kunststoffschlauch eingeleitet wird, um eine Durchmischung zu erzielen.

**Claims**

1. Apparatus for measuring of the quantity of blood flowing in a plastic hose by means of ultrasonic, having an emitter (1), a sampling device (2) including a two-piece housing with a recess (17) into which the plastic hose is insertable, and two oscillator elements (3, 4) for transmitting a signal corresponding to the output signal of the emitter (1) into the blood flowing through the plastic hose and for generating a signal proportional to the velocity of flow and an analyzer including a receiver (5) having a selective high frequency amplifier (6) and a subsequently added demodulator (7), a controlled amplifier (9) and a signal shaper (10, 11) and which analyzer is connected to the sampling device for generation of an output signal which is proportional to the quantity of flow, characterized in that the emitter (1) is designed for generating of an output signal having a frequency of 95 MHz and which emitter having a temperature-compensated circuitry, the oscillator elements (3, 4) are arranged within the sampling device such that in each case at least one surface area of the oscillator elements (3, 4) forms a contact surface for the hose by means of the surface of the recess (17), the high frequency amplifier (6) of the receiver (5) together with the demodulator is designed such that the proportion of intelligence signal and disturbance signal amounts to at least 2 : 1 and the signal shaper (8) includes means (10, 11; 13) for transforming the signal which is proportional to the velocity of flow to a signal which is proportional to the quantity of flow.

2. Apparatus according to claim 1, characterized in that said means include a demodulator (10) for transforming the low frequency signal to an analogous direct current signal and a calibrating amplifier (11).

3. Apparatus according to claim 1, characterized in that the sampling device (2) includes a body (15) having an elongated, recess (17) into which a part of the hose is insertable and includes further a cover (16) for locking the hose part within the recess (17).

4. Apparatus according to claim 1, characterized in that the oscillator elements (3, 4) extend laterally to the longitudinal axis of the recess (17).

5. Apparatus according to claim 1, characterized in that the oscillator elements (3, 4) extend parallel to the longitudinal axis of the recess (17), whereby the surfaces facing the recess form an angle of at least 900.

6. Apparatus according to claim 1, characterized in that the high frequency amplifier (6) of the receiver (5) is provided with quartz filters for improving the selectivity.

7. Apparatus according to claim 1, characterized in that the means include a microprocessor system (13) having lined up analogue to digital converter (12).

8. Apparatus according to claim 7, characterized in that a digital to analogue converter (14) is subsequently added to the microprocessor system (13).

9. Blood-donor device having an apparatus according to claim 1, characterized by a first bag (20) for receiving the donated blood, a second bag (21) comprising a coagulation preventing agent, an arrangement of hoses (22, 23 to 25) connectable on the one hand to the donator and on the other hand to the first and second bag (20, 21), a peristaltic pump (26) for supplying the coagulation preventing agent, connectable to the arrangement of hoses and a control device (27 to 30) for supplying a quantity of the coagulation preventing agent in response to the quantity of blood measured by the apparatus.

10. Device according to claim 9, characterized in that the connection organ (22) is provided such that the coagulation preventing agent supplied from the peristaltic pump (26) is introduced against the stream of the blood within the first plastic hose in order to achieve an intermixture.


**Revendications**

1. Appareil pour la mesure, aux ultrasons, du débit de sang s'écoulant dans un tuyau souple en matière plastique, comprenant un émetteur (1), une sonde (2) qui, en vue d'introduire dans le sang s'écoulant à travers ce tuyau un signal correspondant au signal de sortie de l'émetteur (1) et de produire un signal proportionnel à la vitesse de l'écoulement, comporte un boîtier en deux parties, présentant un évidement (17) dans lequel peut se placer le tuyau souple en matière plastique, et deux éléments oscillants (3, 4), et un dispositif d'exploitation qui, en vue de produire un signal de sortie proportionnel au débit d'écoulement, comporte un récepteur (5), comprenant un amplificateur H.F. sélectif (6) et un démodulateur (7) connecté en aval, un amplificateur de réglage (9) et un circuit de mise en forme de signaux (10, 11), ce dispositif d'exploitation étant raccordé à la sonde, l'appareil étant caractérisé en ce que l'émetteur (1) est agencé de façon à produire un signal de sortie présentant une fréquence de 9,5 MHz et comporte une structure de circuit à compensation de température, en ce que les éléments oscillants (3, 4) sont disposés dans la sonde de façon qu'au moins une section de surface de chacun de ces éléments oscillants (3, 4) forme avec la surface de l'évidement (17) une surface d'appui pour le tuyau souple, en ce que l'amplificateur H.F. (6) du récepteur (5) est dimensionné, de même que le démodulateur, de façon que le rapport signal/bruit vaille au moins 2: 1 et en ce que le circuit de mise en forme de signaux (8) comporte des moyens (10, 11; 13) permettant de convertir le signal proportionnel à la vitesse d'écoulement en un signal proportionnel au débit d'écoulement

2. Appareil suivant la revendication 1, caractérisé en ce que lesdits moyens comprennent un démodulateur (10), permettant de convertir le signal basse fréquence en un signal analogique de tension continue, et un amplificateur de calibrage (11).

3. Appareil suivant la revendication 1, caractérisé en ce que la sonde (2) comprend un corps (15) présentant un évidement allongé (17) dans lequel peut se placer un tronçon du tuyau souple, et un couvercle (16) permettant de fixer ce tronçon de tuyau souple dans cet évidement (17).

4. Appareil suivant la revendication 1, caractérisé en ce que les éléments oscillants (3, 4) sont disposés transversalement à l'axe longitudinal de l'évidement (17).

5. Appareil suivant la revendication 1, caractérisé en ce que les éléments oscillants (3, 4) sont disposés parallèlement à l'axe longitudinal de l'évidement (17), leurs surfaces tournées vers cet évidement faisant entre elles un angle d'au moins 90°.

6. Appareil suivant la revendication 1, caractérisé en ce que l'amplificateur H.F. (6) du récepteur (5) est pourvu de filtres piézoélectriques en vue d'élever la sélectivité.

7. Appareil suivant la revendication 1, caractérisé en ce que lesdits moyens comprennent un système à microprocesseur (13) comportant, connecté en amont, un convertisseur analogique/numérique (12).

8. Appareil suivant la revendication 7, caractérisé en ce qu'un convertisseur numérique/analogique (14) est connecté en aval du système à microprocesseur (13).

9. Appareillage distributeur de sang comprenant un appareil suivant la revendication 1, caractérisé par une première poche (20) destinée à recueillir le sang distribué et une seconde poche (21) qui contient un agent anticoagulant, par un agencement de tuyaux souples (22, 23 à 25) qui peut se brancher d'une part sur le distributeur et d'autre part sur la première et la seconde poches (20, 21), par une pompe péristaltique (26) permettant d'envoyer de l'agent anticoagulant et qui peut être montée sur l'agencement de tuyaux souples et par un dispositif de commande (27 à 30) qui, en fonction du débit du sang distribué qui est mesuré par l'appareil, permet d'envoyer dans ce sang distribué un certain débit d'agent anticoagulant.

10. Appareillage suivant la revendication 9, caractérisé en ce que l'organe de raccordement (22) est disposé de façon que l'agent anticoagulant refoulé par la pompe péristaltique (26) soit introduit dans le premier tuyau souple en matière plastique à contre-courant du sang, afin de réaliser un mélange intime.

Fig. 5

Fig. 4

Fig. 3

Fig. 1

Analog-Signal
mV / $\frac{ml}{min}$

Kalibrierung
Bufferverst.

DF-Demodulator

Regelverstärker

AM-Demodulator

HF-Sender

HF-Verstärker

Fig. 2

EP 0 087 552 B1

Fig.6

**29** Sicherheits- schaltkreis

**31** Spannungs- versorgungsteil

**32**

**33**

**34**

**35**

**27** Pumpensteuer- schaltkreis

Durchfluss- messvorrichtung

400
ml 450
500

**36**

**30**

**20**

**21**

**28**

**24**

**23**

**2**

**22**

**25**

**26**

EP 0 087 552 B1